# FASCICULE DE BREVET EUROPEEN

(11) **EP 2 026 830 B1**
(45) Date de publication et mention de la délivrance du brevet: **27.06.2012**
(21) Numéro de dépôt: 07765933.2
(22) Date de dépôt: 03.05.2007
(51) Int. Cl.: A61K 38/17, C07K 14/47, C07K 14/75

(54) **PEPTIDES MODULATEURS DE L'ACTIVATION DES MACROPHAGES, UTILISABLES POUR LE TRAITEMENT DE LA POLYARTHRITE RHUMATOÏDE**
DIE MAKROPHAGENAKTIVITÄT MODLUIERENDE PEPTIDE ZUR BEHANDLUNG VON RHEUMATOIDER ARTHRITIS
PEPTIDES MODULATING THE ACTIVITY OF MACROPHAGES, USEABLE FOR THE TREATMENT OF RHEUMATOID ARTHRITIS

(30) Priorité: 03.05.2006 FR 0603956
(43) Date de publication de la demande: 25.02.2009
(73) Titulaire: Université Paul Sabatier, 31062 Toulouse Cedex 9 (FR)
(72) Inventeur: CLAVEL, Cyril, F-31830 Plaisance Du Touch (FR); SEBBAG, Mireille, F-31400 Toulouse (FR); NOGUEIRA, Maria Leonor, F-31000 Toulouse (FR); SERRE, Guy, F-31100 Toulouse (FR)
(74) Mandataire: Vialle-Presles, Marie José
(86) Numéro de dépôt international: PCT/FR2007/000758
(87) Numéro de publication internationale: WO 2007/125226

(56) Documents cités:
- WO-A-01/02437
- WO-A-02/48180
- WO-A-02/48181
- WO-A-2006/048556
- WO-A2-99/34819
- VAN GAALEN FLORIS ET AL: "The devil in the details: The emerging role of anticitrulline autoimmunity in rheumatoid arthritis" JOURNAL OF IMMUNOLOGY, vol. 175, no. 9, novembre 2005 (2005-11), pages 5575-5580, XP002408403 ISSN: 0022-1767
- FOURNIER ET AL: "Where Do T Cells Stand in Rheumatoid Arthritis?" JOINT BONE SPINE, ELSEVIER, PARIS, FR, vol. 72, no. 6, décembre 2005 (2005-12), pages 527-532, XP005221943 ISSN: 1297-319X
- SEBBAG M ET AL: "Clinical and pathophysiological significance of the autoimmune response to citrullinated proteins in rheumatoid arthritis" JOINT BONE SPINE, ELSEVIER, PARIS, FR, vol. 71, no. 6, novembre 2004 (2004-11), pages 493-502, XP004678687 ISSN: 1297-319X
- VOSSENAAR ERIK R ET AL: "Rheumatoid arthritis specific anti-Sa antibodies target citrullinated vimentin" ARTHRITIS RESEARCH, CURRENT SCIENCE, LONDON,, GB, vol. 6, no. 2, 5 février 2004 (2004-02-05), pages R142-R150, XP021011474 ISSN: 1465-9905 cité dans la demande

## Description

La présente Invention est relative à un modèle *in vitro* d'activation des macrophages, induite par des complexes immuns entre des IgG spécifiques de la polyarthrite rhumatoïde (ci après abrégée en « PR ») et leurs cibles citrullinées, et à son utilisation pour identifier des molécules possédant des propriétés de modulation de l'inflammation.

La polyarthrite rhumatoïde est le plus fréquent des rhumatismes inflammatoires chroniques. Il s'agit d'une maladie auto-immune, et le sérum des patients atteints contient des auto-anticorps dont certains sont spécifiques, et peuvent constituer un marqueur de cette maladie, permettant son diagnostic même à des stades précoces. Des recherches ont donc été effectuées en vue d'identifier des antigènes reconnus par ces anticorps, afin d'en obtenir des préparations purifiées utilisables dans des techniques classiques de diagnostic immunologique .

Il a été montré que des auto-anticorps (IgG) spécifiques de la PR reconnaissaient différents variants isoélectriques de la (pro)filaggrine (pour revue, cf. par exemple SERRE et VINCENT, In : Autoantibodies, PETER and SHOENFELD Eds, Elsevier Science Publishers, 271-276, 1996). Ces auto-anticorps ont pour cette raison été dénommés : « auto-anticorps anti-filaggrine (AAF) ». La Demande EP 0 511 116 décrit la purification et la caractérisation d'antigènes de la famille des filaggrines reconnus par ces anticorps, et leur utilisation pour le diagnostic de la polyarthrite rhumatoïde.

Ultérieurement, les épitopes de la filaggrine reconnus par les AAF ont été identifiés comme des régions de la molécule de filaggrine porteuses de résidus citrullyl, résultant de la transformation des résidus arginyl par une peptidylarginine désiminase (Girbal-Neuhauser E et al., J Immunol, 162, 585-94, 1999 ; Schellekens GA et al., J Clin Invest, 101, 273-81, 1998). L'analyse de divers peptides synthétiques dérivés de la séquence de la filaggrine humaine a montré que la désimination (citrullination) était nécessaire à la constitution des épitopes reconnus par les AAF, qui sont aujourd'hui également appelés auto-anticorps anti-peptides citrullinées (AAPC).

De très nombreux peptides citrullinés spécifiquement reconnus par des AAPC, et utilisables pour le diagnostic de la PR ont été obtenus à partir de la filaggrine (Demande EP 0 929 669, Demande EP 1 475 438), ainsi que d'autres protéines citrullinées, parmi lesquelles on citera notamment la fibrine (Demande WO 01/02437) et la vimentine (Vossenaar ER et al., Arthritis Res Ther 2004;6:R142-R150).

Parallèlement, il a été montré que les AAPC sont sécrétés par les plasmocytes du tissu synovial (Masson-Bessière C et al., Clin Exp Immunol, 119, 544-52, 2000) et sont spécifiquement dirigés contre des formes citrullinées des chaînes α et β de fibrine présentes dans ce tissu (Masson-Bessière C et al., J Immunol, 166, 4177-84, 2001; Demande PCT WO 01/02437).

Il a été suggéré que la formation de complexes immuns (ci après abrégés en « CI ») entre les AAPC et les épitopes citrullinés spécifiques de ceux-ci, présents dans le tissu synovial, jouerait un rôle dans la pathogenèse de la polyarthrite rhumatoïde et notamment dans le déclenchement et le maintien de la réaction inflammatoire. Van Gaalen et al. (Journal of Immunology, 175, 5575, 2005) proposent un modèle pour expliquer le rôle des AAPC in vivo dans la PR, dans lequel des complexes immuns formés entre les AAPC et des protéines citrullinées présentes dans le tissu synovial activent le complément. Cette activation du complément déclencherait la libération de C5a (fragment du complément qui est connu comme l'un des médiateurs prépondérants dans l'inflammation). En outre, la voie du complément permettrait d'attirer et d'activer des granulocytes, monocytes, macrophages et mastocytes sur le lieu de l'inflammation.

Fournier (Joint Bone Spine, 72, 527, 2005) décrit le rôle des cellules T dans le développement de la PR. Il rapporte que dans le cas de l'arthrose collagène-induite (CIA), modèle d'étude expérimental de la PR chez la souris, des cytokines pro-inflammatoires (notamment TNF-α et IL-1β) sont produites par les macrophages en présence de cellules T activées présentant un phénotype Th1, et que la neutralisation de ces cytokines pro-inflammatoires est efficace pour réduire la sévérité de la CIA.

Les inventeurs ont mis au point un modèle *in vitro* de l'activation des macrophages, induite par l'interaction entre les CI formés à partir des AAPC et de leur épitopes citrullinés, et les macrophages.

Ce modèle, qui mime les conditions physiopathologiques du tissu synovial rhumatoïde, met en oeuvre l'évaluation de l'activation des macrophages, après mise en contact de ceux-ci *in vitro* avec des CI formés par des AAPC et des polypeptides citrullinés reconnus par lesdits AAPC.

L'activation des macrophages, qui stimule la production de cytokines pro-inflammatoires, peut être évaluée par le dosage d'une ou plusieurs de ces cytokines (par exemple TNF-α, IL-1β, IL-6, IL-8, GM-CSF, IL-15...) dans les surnageants de culture de macrophages, après mise en contact de ceux-ci avec lesdits CI.

Ce modèle est particulièrement adapté à l'évaluation des propriétés de modulation de l'inflammation d'une molécule, et notamment de ses propriétés anti-inflammatoires.

Il est notamment utilisable pour l'identification *in vitro* d'agents pharmacologiques susceptibles d'avoir un effet antagoniste de l'activation des macrophages par les CI, soit en s'opposant à la formation de complexes immuns entre les AAPC et les antigènes citrullinés reconnus par lesdits AAPC, soit en s'opposant à la liaison desdits complexes aux macrophages.

L'évaluation des propriétés de modulation de l'inflammation d'une molécule à tester peut s'effectuer simplement, en comparant la production d'une ou plusieurs cytokines pro-inflammatoires dans les surnageants de culture de macrophages activés par les CI comme décrit ci-dessus, en l'absence de la molécule à tester, et en présence de celle-ci.

La présente invention a pour objet un procédé d'évaluation *in vitro,* des propriétés de modulation de l'inflammation d'une molécule, caractérisé en ce qu'il comprend :
a) la mise en présence d'auto-anticorps anti-peptide citrulliné (AAPC) avec un antigène peptidique citrulliné réactif avec lesdits AAPC, dans des conditions permettant la formation de complexes immuns entre lesdits AAPC et ledit antigène citrulliné ;
b) la mise en présence des complexes immuns formés en a) avec des macrophages, dans des conditions permettant l'activation desdits macrophages par lesdits complexes immuns, et la détermination de la quantité d'au moins une cytokine pro-inflammatoire produite par lesdits macrophages ;
c) la répétition de l'étape a) et de l'étape b) l'une et/ou l'autre desdites étapes étant répétée(s) en présence de la molécule à tester ;
d) la comparaison de la quantité de cytokine(s) pro-inflammatoire(s) produite en l'absence de la molécule à tester et en présence de celle-ci.

Avantageusement, l'étape a) est suivie d'une étape d'élimination des IgG qui ne sont pas impliquées dans la formation de complexes immuns avec l'antigène citrulliné. Il s'agit par exemple des IgG libres (n'ayant pas réagi avec l'antigène citrulliné à l'issue de l'étape a)) ou le cas échéant, des IgG éventuellement impliquées dans la formation de complexes immuns avec la molécule à tester.

Dans ce cas l'antigène citrulliné sera immobilisé sur un support solide, tel que des plaques de microtitration ou des billes magnétiques, afin de permettre de réaliser facilement cette élimination par rinçage.

Les AAPC utilisables pour la mise en oeuvre du procédé conforme à l'invention sont par exemple des IgG obtenues à partir d'un sérum, ou avantageusement d'un mélange de sérums, de patient(s) atteint(s) de polyarthrite rhumatoïde, et présentant une réaction positive à un ou, de préférence plusieurs, tests de référence de détection des AAPC (par exemple le test d'immunofluorescence indirecte sur cryocoupes d'oesophage de rat décrit par Vincent C et al., Ann Rheum Dis 48, 712-22, (1989) ou le test ELISA sur fibrinogène humain citrulliné décrit par Chapuy-Regaud S et al., Clin Exp Immunol, 139:542-50, 2005). Avantageusement lesdites IgG peuvent en outre être purifiées par chromatographie à l'aide d'un antigène citrulliné tel que le fibrinogène citrulliné, la filaggrine citrullinée, la vimentine citrullinée, etc. On peut aussi utiliser éventuellement des AAPC monoclonaux, ou produits par recombinaison génétique.

L'antigène citrulliné utilisé pour la formation des CI avec les AAPC, peut être tout polypeptide ou mélange de polypeptides citrulliné(s) (naturel(s) ou obtenu(s) par recombinaison génétique ou synthèse chimique puis citrullination *in vitro*) capable(s) de réagir avec lesdits AAPC. Il peut s'agir notamment de la filaggrine citrullinée (Demande EP 0 511 116, Demande EP 0 929 669, Demande EP 1 475 438), de la fibrine ou du fibrinogène citrulliné (Demande WO 01/02437), de la vimentine citrullinée (Hill A et al., The Journal of Immunology, 2003, 171: 538-541 ; Vossenaar ER et al., Arthritis Res 2000, 2:429-432), ou de fragments citrullinés de ces protéines capables de réagir avec la préparation d'AAPC utilisée. L'homme du métier peut vérifier aisément, par des tests immunologiques de base, la réactivité d'un antigène citrulliné, quel qu'il soit, avec une préparation d'AAPC.

Avantageusement, ledit antigène citrulliné comprend un polypeptide citrulliné dérivé de la fibrine, choisi parmi ceux définis ci-dessous, ou un mélange de deux ou plus de ces polypeptides.

Préférentiellement, les macrophages utilisés sont des macrophages de mammifère, notamment des macrophages d'origine humaine, qui peuvent être obtenus par exemple à partir de monocytes isolés à l'aide d'un anticorps anti-CD14, et différenciés en macrophages par culture en présence de M-CSF (macrophage colony stimulating factor).

Pour mesurer l'activation des macrophages on peut doser une (ou éventuellement plusieurs) cytokine(s) pro-inflammatoire(s), sélectionnée(s) de préférence parmi le TNF-α (Tumor Necrosis Factor-α), l'interleukine 1β (IL-1β), l'interleukine 6 (IL-6), l'interleukine 8 (IL-8), le GM-CSF (Granulocyte Macrophage-Colony Stimulating Factor), et l'interleukine 15 (IL-15). Une cytokine pro-inflammatoire préférée est le TNF-α, abondamment produit par les macrophages du tissu synovial rhumatoïde et qui joue un rôle majeur dans l'inflammation et la destruction articulaires (Feldmann M et al., Annu Rev Immunol 14:397-440, 1996).

Les concentrations en AAPC et en antigène peptidique citrulliné permettant une formation optimale du complexe immun, et une activation optimale des macrophages peuvent dépendre notamment de la préparation d'AAPC, et de celle d'antigène peptidique utilisées. L'homme du métier peut aisément déterminer les conditions réactionnelles optimales en procédant à de simples essais de calibrage de routine, comme ceux décrits dans les exemples ci-après.

L'invention a en outre pour objet un nécessaire (ou trousse ou kit) pour la mise en oeuvre d'un procédé conforme à l'invention, caractérisé en ce qu'elle comprend un antigène peptidique citrulliné optionnellement immobilisé sur un support solide, et des auto-anticorps anti-peptide citrulliné (AAPC) capables de former un complexe immun avec ledit antigène.

Ledit nécessaire peut comprendre en outre des moyens de dosage d'une ou plusieurs cytokine(s) pro-inflammatoire (s) .

Optionnellement, ledit nécessaire comprend en outre du M-CSF et un moyen de sélection de monocytes, tel qu'un support revêtu d'anticorps anti-CD14.

La présente invention a également pour objet un procédé de sélection de molécules modulatrices de l'inflammation, caractérisé en ce qu'il comprend :
- la mise en oeuvre, avec chacune des molécules à tester, d'un procédé d'évaluation des propriétés de modulation de l'inflammation de ladite molécule, tel que défini ci-dessus,
- la sélection des molécules capables de modifier la quantité de cytokine(s) pro-inflammatoire(s) produite(s) par les macrophages.

Selon un mode de mise en oeuvre préféré d'un procédé de sélection conforme à l'invention, il s'agit d'un procédé de sélection de molécules anti-inflammatoires, et il comprend la sélection des molécules capables de diminuer la quantité de cytokine(s) pro-inflammatoire(s) produite(s) par les macrophages.

Des molécules anti-inflammatoires capables de diminuer la quantité de cytokine(s) pro-inflammatoire(s), produite(s) par les macrophages lors de la mise en oeuvre d'un procédé conforme à l'invention sont utilisables notamment pour la préparation de médicaments destinés au traitement de maladies inflammatoires impliquant des complexes immuns associant des AAPC et des antigènes citrullinés, et en particulier de la polyarthrite rhumatoïde.

Dans ce cadre, la présente invention a également pour objet un peptide citrulliné, capable de diminuer la quantité de TNF-α produit par les macrophages lors de la mise en oeuvre d'un procédé conforme à l'invention, pour l'utilisation comme médicament, notamment pour le traitement de maladies inflammatoires, et en particulier de la polyarthrite rhumatoïde.

Ledit peptide est sélectionné dans le groupe comprenant les peptides suivants :
- un peptide défini par la séquence X₁PAPPPISGGGYX₂AX₃ (SEQ ID NO: 1) dans laquelle X₁ et X₂, représentent chacun un résidu citrullyl ou un résidu arginyl, et X₃ est un résidu citrullyl, ou un peptide de 5 à 25 acides aminés, de préférence de 10 à 20 acides aminés, comprenant un fragment d'au moins 5 acides aminés consécutifs de ladite séquence contenant ledit résidu citrullyl X₃ ;
- un peptide défini par la séquence GPX₁VVEX₂HQSACKDS (SEQ ID NO: 2) dans laquelle X₁ et X₂ représentent chacun un résidu citrullyl ou un résidu arginyl, et au moins l'un des résidus X₁ ou X₂ est un résidu citrullyl, ou un peptide de 5 à 25 acides aminés, de préférence de 10 à 20 acides aminés, comprenant un fragment d'au moins 5 acides aminés consécutifs de ladite séquence contenant ledit ou lesdits résidu(s) citrullyl.

Avantageusement, ledit peptide est sélectionné dans le groupe comprenant les peptides suivants :
- un peptide défini par la séquence SEQ ID NO: 1 dans laquelle X₁ et X₂ sont des résidus arginyl et X₃ est un résidu citrullyl, ou un peptide de 5 à 25 acides aminés comprenant un fragment d'au moins 5 acides aminés consécutifs de ladite séquence contenant ledit résidu citrullyl ;
- un peptide défini par la séquence SEQ ID NO: 1 dans laquelle X₁ est un résidu arginyl et X₂ et X₃ sont des résidus citrullyl, ou un peptide de 5 à 25 acides aminés comprenant un fragment d'au moins 5 acides aminés consécutifs de ladite séquence contenant lesdits résidus citrullyl ;
- un peptide défini par la séquence SEQ ID NO: 1 dans laquelle X₂ est un résidu arginyl et X₁ et X₃ sont des résidus citrullyl, ou un peptide de 16 à 25 acides aminés comprenant lesdits résidus citrullyl ;
- un peptide défini par la séquence SEQ ID NO: 1 dans laquelle X₁, X₂, et X₃ sont des résidus citrullyl, ou un peptide de 16 à 25 acides aminés comprenant lesdits résidus citrullyl ;
- un peptide défini par la séquence SEQ ID NO: 2 dans laquelle X₁ est un résidu arginyl et X₂ est un résidu citrullyl, ou un peptide de 5 à 25 acides aminés comprenant un fragment d'au moins 5 acides aminés consécutifs de ladite séquence contenant ledit résidu citrullyl ;
- un peptide défini par la séquence SEQ ID NO: 2 dans laquelle X₁ est un résidu citrullyl et X₂ est un résidu arginyl, ou un peptide de 5 à 25 acides aminés comprenant un fragment d'au moins 5 acides aminés consécutifs de ladite séquence contenant ledit résidu citrullyl ;
- un peptide défini par la séquence SEQ ID NO: 2 dans laquelle X₁ et X₂ sont des résidus citrullyl, ou un peptide de 5 à 25 acides aminés comprenant un fragment d'au moins 5 acides aminés consécutifs de ladite séquence contenant lesdits résidus citrullyl.

Les peptides citrullinés utilisables comme médicament conformément à l'invention englobent également des dérivés des peptides SEQ ID NO: 1 à 5 ou des fragments de ceux-ci définis ci-dessus, lesdits dérivés portant des modifications destinées à améliorer leur reconnaissance par les AAPC : à titre d'exemples de tels dérivés, on citera : des peptides cyclisés ; des peptides de type *rétro,* dans lesquels des acides L-aminés sont enchaînés selon une séquence inverse de celle du peptide à reproduire ; des peptides de type *rétro-inverso,* constitués par des acides aminés de la série D (au lieu des acides aminés de la série L des peptides naturels) enchaînés selon une séquence inverse de celle du peptide à reproduire.

Très avantageusement, il s'agit de peptides dans lesquels la fonction carboxyle (COOH) terminale est remplacée par une fonction carboxamide (CONH₂) . Dans ce cadre, des peptides particulièrement préférés sont ceux dans lesquels le résidu C-terminal est un résidu citrullyl dont la fonction carboxyle est remplacée par une fonction carboxamide, par exemple dans le cas du peptide défini par la séquence SEQ ID NO : 1, lorsqu'au moins X₃ est un résidu citrullyl.

Les peptides citrullinés utilisables comme médicament conformément à l'invention englobent également des dérivés des peptides SEQ ID NO: 1 à 5, ou des fragments de ceux-ci tels que définis ci-dessus, lesdits dérivés portant des modifications destinées à faciliter leur synthèse et/ou à améliorer leur stabilité. A titre d'exemple de tels dérivés, on citera les peptides incluant des acides aminés dont les groupements carboxyl sont estérifiés ou transformés en groupements amide et/ou des acides aminés dont un groupement aminé est alkylé, par exemple méthylé ou acétylé. Les groupements amine et carboxyl des peptides peuvent être présents sous forme du sel correspondant à la base ou à l'acide.

A partir des peptides citrullinés décrits ci-dessus, il est aussi possible d'obtenir des peptides mimotopes comprenant au moins un résidu citrullyle (peptide mimotope citrulliné), également utilisables comme médicaments conformément à l'invention.

Ces peptides mimotopes peuvent être obtenus en synthétisant des banques de peptides citrullinés dont les séquences sont définies à partir de celles des peptides SEQ ID n° 1 à 5, utilisés dans ce cadre comme "peptides modèles", et en mettant en oeuvre le procédé conforme à l'invention, pour évaluer les propriétés anti-inflammatoires de ces peptides.

De préférence, ces banques de peptides sont réalisées par synthèse de différents peptides de taille comprise entre 10 et 20, de préférence entre 12 et 17 acides aminés, en particulier 15 acides aminés. Chacun de ces peptides conserve au moins 2, de préférence au moins 4, avantageusement au moins 6, de manière particulièrement préférée au moins 8, et très avantageusement au moins 10 acides aminés, dont au moins un résidu citrullyl, de la séquence du peptide modèle choisi, aux mêmes positions que sur ledit peptide modèle, les autres positions étant variables.

Des méthodes de synthèse de peptides mimotopes sont bien connues en elles-mêmes. On se référera par exemple au chapitre 6 de "Chemical approaches to the synthesis of peptides and proteins", Paul Lloyd-Williams, Fernando Albericio and Ernest Giralt, CRC Press New York, 1997, "Peptides libraries", pages 237-270.

Conformément à l'invention, les peptides citrullinés définis ci-dessus peuvent être utilisés seuls, en combinaison entre eux, ou, le cas échéant avec d'autres peptides citrullinés.

La présente invention a ainsi pour objet des compositions pharmaceutiques, caractérisées en ce qu'elles comprennent, en tant que principe actif, au moins un peptide citrulliné tel que défini ci-dessus.

Des compositions conformes à l'invention peuvent par exemple associer entre eux différents peptides citrullinés choisis parmi ceux définis ci-dessus, ou bien peuvent associer un ou plusieurs desdits peptides, avec un ou plusieurs peptides citrullinés dérivés notamment de la filaggrine.

Selon un mode de réalisation préféré d'une composition pharmaceutique conforme à l'invention, elle comprend au moins un peptide de séquence SEQ ID NO: 1, et au moins un peptide de séquence SEQ ID NO: 2, tels que définis ci-dessus, et éventuellement elle peut comprendre en outre un peptide de séquence SEQ ID NO: 3 et/ou un peptide de séquence SEQ ID NO: 4 et/ou un peptide de séquence SEQ ID NO: 5, tels que définis ci-dessus.

Des compositions pharmaceutiques conformes à l'invention peuvent comprendre en outre des excipients ou additifs habituellement utilisés en pharmacie.

L'invention sera mieux comprise à l'aide des figures et des exemples non-limitatifs qui suivent et qui sont présentés ici dans un but illustratif.

### FIGURES

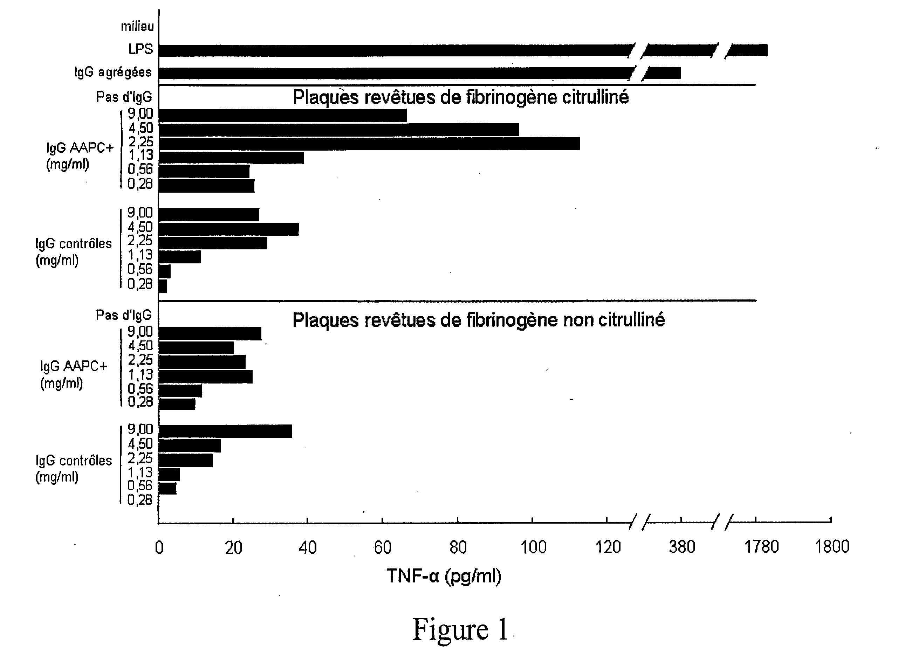

**Figure 1** **:** Mesure du taux de TNF-α (pg/ml) sécrété par les macrophages en présence de fibrinogène citrulliné ou non citrulliné, et de différentes concentrations (0 ; 0,28 ; 0,56 ; 1,13 ; 2,25 ; 4,50 et 9,00 mg/ml) d'AAPC (IgG AAPC+), ou d'IgG contrôles. Le taux de TNF-α est également mesuré avec les macrophages mis en présence d'IgG agrégées par la chaleur (IgG agrégées), de Lipopolysaccharide bactérien (LPS) ou laissés en milieu macrophage-SFM seul (milieu).

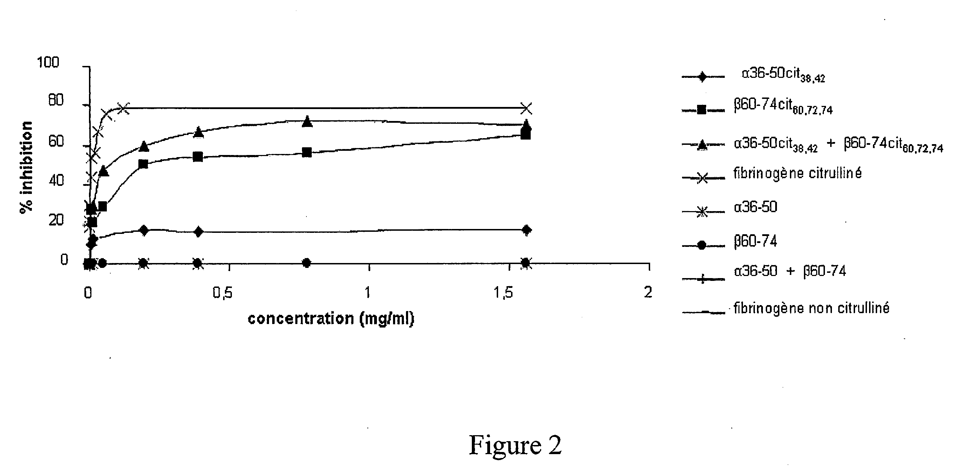

**Figure 2** : Effet de concentrations croissantes (de 0,006 à 1,56 mg/ml) en fibrinogène citrulliné (croix) ou non citrulliné (trait), en peptide α36-50_{Cit38}, ₄₂ (losange), en peptide α36-50 (croix à 6 branches), en peptide β60-74Cit_{60, 72, 74} (carré), en peptide β60-74 (rond), en mélange de peptides α36-50Cit_{38, 42} et β60-74Cit_{60, 72, 74} (triangle) ou de peptides α36-50 et β60-74 (tiret vertical) sur le pourcentage d'inhibition de la réactivité des AAPC+ vis-à-vis du fibrinogène citrulliné.

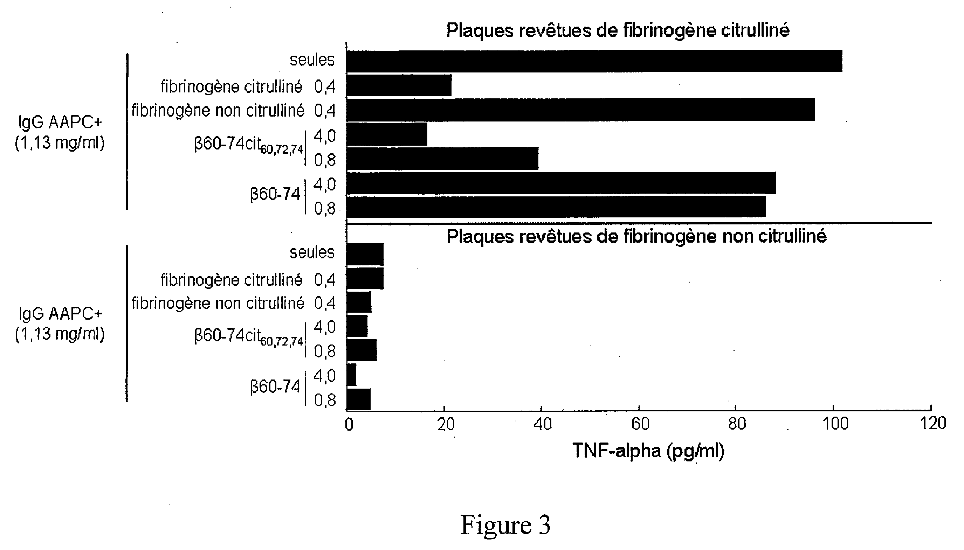

**Figure 3** : Mesure du taux de TNF-α (pg/ml) sécrété par les macrophages mis en présence de fibrinogène citrulliné ou non citrulliné (contrôle) et de 1,13 mg/ml d'IgG AAPC+ et en présence ou absence des compétiteurs suivants : fibrinogène citrulliné ou non citrulliné (4,0 mg/ml), peptide β60-74Cit_{60, 72, 74} ou peptide β60-74 (0,8 ou 4,0 mg/ml).

### EXEMPLE 1 : EVALUATION IN VITRO DE L'EFFET PRO-INFLAMMATOIRE DE COMPLEXES IMMUNS ASSOCIANT FIBRINOGENE CITRULLINE ET AAPC.

Le présent exemple concerne la réalisation du modèle de stimulation *in vitro* de macrophage par des complexes immuns formés à partir d'AAPC et d'épitopes citrullinés réactifs avec lesdits AAPC.

Des monocytes sont purifiés à partir de cellules mononucléées sanguines d'individus sains à l'aide d'un anticorps anti-CD14 couplé à des billes magnétiques. Ces monocytes sont différenciés en macrophages par culture durant 7 jours en présence de M-CSF puis stimulés par des CI immobilisés .

Ces derniers sont reconstitués en faisant réagir des AAPC purifiés à partir de sérums de patients atteints de PR, sur du fibrinogène humain citrulliné adsorbé en fond de plaque de culture. L'activation des macrophages est appréciée par leur sécrétion de TNF-α, dosé dans les surnageants de culture après 24h de contact.

Les détails techniques qui ont permis de réaliser ce modèle de stimulation des macrophages sont décrits ci-après.

### Préparation des macrophages:

A partir d'échantillons de sang ou de concentrés leucocytaires d'individus sains (Établissement Français du Sang, Toulouse, France) les cellules mononucléées sont séparées des débris et autres cellules sanguines par centrifugation pendant 20 min à 1200 g sur Ficoll (Ficoll 400®, Biocoll isoton separating solution, 1,077 g/ml, Biochrom AG, Berlin, Allemagne) à raison de 15ml de Ficoll pour 30ml de suspension cellulaire diluée au ½ dans du PBS (Phosphate Buffer Saline) à pH 7,4 contenant 0,1% d'albumine sérique (BSA, Sigma-Aldrich Chimie, St Quentin Fallavier, France) et 0,6% de Citrate de Sodium (cette solution sera par la suite dénommée PBNaCit). Après 2 lavages par 25ml de PBNaCit, les cellules viables sont énumérées à l'aide d'une cellule de comptage après coloration au bleu Trypan 0,4%. Un tri positif des cellules de la lignée monocytaire (exprimant le marqueur CD14) est effectué à l'aide d'un anticorps anti-CD14 humain couplé à des billes magnétiques (CD14 MicroBeads, Miltenyi Biotec, Paris, France). Un mélange respectant une proportion de 140µl d'anticorps anti-CD14 et 1200µl de PBNaCit pour 140x10⁶ cellules mononucléées est incubé pendant 15 minutes à 4°C. Après lavage par 25 ml de PBNaCit, la suspension cellulaire est chargée sur une colonne contenant des billes ferromagnétiques (colonne MS, Miltenyi Biotec) conditionnée par 1ml de PBNaCit et placée dans un champ magnétique. Après 3 lavages par 500µl de PBNaCit permettant d'éliminer les cellules n'exprimant pas CD14, la colonne est écartée du champ magnétique et les cellules marquées par l'anticorps anti-CD14 sont chassées par 500µl de PBNaCit. Les cellules viables sont énumérées à l'aide d'une cellule de comptage après coloration au bleu Trypan 0,4%. Les monocytes sont ensuite mis en culture à raison de 10⁶ cellules viables/ml en milieu macrophage-SFM (milieu dépourvu de sérum conçu pour la culture de monocytes et macrophages périphériques humains, Gibco, Invitrogen, Cergy Pontoise, France) additionné de sérum de veau foetal (10%, v/v, Biowest, Nuaillé, France) et de M-CSF *(Macrophage Colony Stimulating Factor)* à 100 ng/ml (Peprotech, Levallois-Perret, France) dans des puits en téflon, durant 7 jours à 37°C sous atmosphère contenant 5% de CO₂. Au terme de cette incubation, on obtient des macrophages. Ceux-ci sont lavés en milieu macrophage-SFM puis les cellules viables sont énumérées à l'aide d'une cellule de comptage après coloration au bleu Trypan 0,4%.

### Citrullination (désimination) de fibrinogène humain

Une préparation commerciale de fibrinogène purifié humain (Calbiochem, Meudon, France) est débarrassée des IgG contaminantes résiduelles par chromatographie d'affinité sur colonne de protéine G (HiTrap Protein G, GE Healthcare, Orsay, France) selon les conditions préconisées par son fabricant. Le fibrinogène est ensuite incubé pendant 2 heures à 37°C dans un tampon Tris-HCl 0,1M pH 7,4, CaCl₂ 10 mM, dithiothréitol 5 mM et en présence ou absence de peptidylarginine désiminase de muscle squelettique de lapin (Sigma-Aldrich) à raison de 7 U d'enzyme/mg de fibrinogène. Les différentes chaînes constitutives du fibrinogène, dissociées suite à l'incubation dans ce tampon réducteur de ponts disulfures, sont ensuite réassociées par dialyse contre du PBS poursuivie jusqu'à réassociation complète des différentes chaînes, contrôlée par PAGE-SDS en conditions non réductrices. On obtient ainsi deux préparations de fibrinogène : citrulliné (incubé en présence d'enzyme) et non citrulliné (incubé en absence d'enzyme).

### Préparation d'IgG humaines agrégées par la chaleur

Les IgG humaines agrégées solubles (ci-après dénommées IgG agrégées) sont préparées par incubation à 63°C pendant 20 minutes d'une solution d'IgG humaines d'individus sains purifiées (Sigma-Aldrich) à 2 mg/ml dans du PBS. Après centrifugation à 12000 g, le surnageant contenant les IgG humaines agrégées solubles est récupéré et utilisé extemporanément.

### Préparation d'un mélange d'IgG AAPC-positif

Un mélange d'IgG AAPC-positif (ci-après dénommé IgG AAPC+) est préparé par isolement de la fraction IgG d'un mélange à parts égales de 38 sérums de patients atteints de PR présentant un titre sérique élevé d'AAPC, par chromatographie d'affinité sur une colonne de protéine G de 5 ml (HiTrap Protein G, GE Healthcare, Orsay, France). Après équilibration de la colonne en tampon KH₂PO₄/K₂HPO₄ 0,02M pH 7,0, 40 ml de mélange de sérums dilués au ¼ dans ce même tampon et passés à travers un filtre de 0,22 µm sont chargés sur la colonne à raison de 2ml par minute. Après lavage de la colonne par 5 volumes de KH₂PO₄/K₂HPO₄ 0,02M pH 7,0 contenant 1M de NaCl, les IgG totales AAPC+ sont éluées en tampon glycine-HCl à 0,2M pH 2,7. Les fractions d'élutions sont immédiatement placées à pH neutre par ajout de Tris 2M. Les fractions les plus riches en IgG sont repérées par suivi de la densité optique à 280 nm (Biophotomètre Eppendorf, Eppendorf, Dominique Dutscher, Brumath, France), regroupées et dialysées contre KH₂PO₄/K₂HPO₄ 0,02M pH 7,0 contenant 0,5 M de NaCl, passées à travers un filtre de 0,22 µm puis aliquotées et stockées à -20°C jusqu'à utilisation. La qualité de la purification est contrôlée par coloration au bleu de Coomassie après électrophorèse sur automate PHAST-system (GE Healthcare, Orsay, France) en gel PAGE-SDS 12,5%.

### Préparation d'un mélange d'IgG AAPC-négatif

Un mélange d'IgG AAPC-négatif (ci-après dénommé IgG contrôles) est préparé par isolement de la fraction IgG d'un mélange à parts égales de 20 sérums humains présentant tous un titre nul après dosage des AAPC par deux tests de référence, immunofluorescence indirecte sur cryocoupes d'oesophage de rat (Vincent C et al., Ann Rheum Dis 48, 712-22, 1989) et ELISA sur fibrinogène humain citrulliné (Chapuy-Regaud S et al., Clin Exp Immunol, 139:542-50, 2005). L'isolement de la fraction IgG est réalisé par chromatographie d'affinité sur une colonne de protéine G exactement selon le protocole utilisé pour la préparation des IgG AAPC+.

### Reconstitution de complexes immuns (CI) associant AAPC et fibrinogène citrulliné:

La reconstitution de CI associant AAPC et fibrinogène citrulliné est réalisée par revêtement en conditions stériles de plaques de culture de 96 puits à fond plat (Nunclon delta, Nunc, Roskilde Danemark) par du fibrinogène humain citrulliné à 10 µg/ml ajouté à raison de 50 µl/puits et incubé une nuit à 4°C. Les plaques sont ensuite saturées en PBS pH 7,4 BSA 2%, pendant 1 heure à 4°C (180 µl/puits). Après 3 lavages en PBS pH 7,4 Tween-20 0,1%, on incube les puits pendant 2 heures à 4°C avec 100 µl d'IgG AAPC+ à différentes concentrations (variant de 9 à 0,28 mg/ml) diluées dans du PBS contant 2% BSA et 2M NaCl. Des contrôles négatifs sont constitués de la même façon en utilisant soit du fibrinogène humain non citrulliné comme antigène revêtu en fond de plaque, soit des IgG contrôles comme anticorps. Des puits uniquement revêtus de fibrinogène humain citrulliné ou non citrulliné et saturés en PBS pH 7,4 BSA 2% sont également utilisés comme contrôles négatifs.

### Stimulation des macrophages:

Après 3 lavages en PBS pH 7,4 Tween-20 0,1%, puis 3 lavages en PBS, on ajoute les macrophages à raison de 50000 cellules viables/puits sous un volume de 200 µl. Après incubation pendant 24 heures à 37°C sous atmosphère contenant 5% de CO₂, on récupère les surnageants (160µl/puits) qui sont rapidement congelés à -20°C jusqu'à analyse. Le contrôle négatif d'activation est réalisé à l'aide de macrophages dans du milieu macrophage-SFM seul. Les macrophages placés sur des IgG agrégées immobilisées au fond des plaques de culture par adsorption passive (à raison de 100 µl d'une solution à 5 µg/ml par puits), ou mis en présence de lipopolysaccharide bactérien (LPS, *Escherichia coli* 055:B5 à 0,5 µg/ml final; Sigma-Aldrich Chimie) sont utilisés comme contrôles de la capacité des macrophages à répondre à un stimulus inducteur de la synthèse de TNF-α.

### Dosage du TNF-α dans les surnageants de culture

Une trousse de dosage par ELISA du TNF-α humain comprenant un anticorps de capture, un anticorps de détection et du TNF-α humain recombinant pour la constitution d'une gamme d'étalonnage de 7,8 à 500 pg/ml a été utilisée pour le dosage de cette cytokine dans les surnageants de culture (Human TNF-alpha BD OptEIA™ ELISA Set, BD Biosciences Pharmingen, Pont-de-Claix, France).

### Résultats:

Les résultats obtenus sont illustrés dans la figure 1. Ces données sont représentatives du résultat de 7 expériences différentes sur un total de 10 expériences conduites avec les macrophages d'individus différents.

L'accrochage non spécifique d'IgG contrôles sur le fibrinogène citrulliné ou non et des IgG AAPC+ sur le fibrinogène non citrulliné permet d'induire un niveau basal de synthèse de TNF-α inférieur à 40 pg/ml. Cette stimulation est nettement majorée lorsque des CI spécifiques sont reconstitués suite à l'interaction des IgG AAPC+ avec le fibrinogène citrulliné, lorsqu'on utilise des concentrations d'IgG AAPC+ supérieures à 1,13 mg/ml. Un maximum de stimulation est observée pour une concentration d'IgG AAPC+ de 2,25 mg/ml et le taux de TNF-α atteint une valeur proche de 120 pg/ml.

Il est à noter que quelles que soient les IgG utilisées, l'induction de TNF-α se majore lorsqu'on utilise des concentrations croissantes d'IgG pour ensuite diminuer de manière également dose-dépendante (effet « courbe en cloche »). Ceci laisse présumer qu'au-delà d'une certaine concentration d'IgG immobilisées des voies exerçant un effet régulateur négatif sur la production de TNF-α sont activées.

Comme attendu, la stimulation par les substituts de CI que constituent les IgG humaines agrégées par la chaleur induit une production importante de TNF-α. Des quantités très importantes de TNF-α sont également produites en réponse au LPS.

Ce modèle cellulaire et moléculaire d'origine entièrement humaine permet de démontrer l'effet pro-inflammatoire de l'interaction entre macrophages et CI associant fibrinogène citrulliné et AAPC, puisque ces complexes reconstitués *in vitro i*nduisent spécifiquement la production de TNF-α par ces cellules (Figure 1). Cette production est en effet nettement accrue par rapport à celle que l'on obtient après mise en contact avec des IgG de spécificité quelconque, probablement immobilisées sur les plaques de culture suite à un accrochage non spécifique au fibrinogène citrulliné ou non. Elle est également dépendante de la quantité d'IgG AAPC+ ajoutées, permettant de définir une concentration optimale favorisant la formation de CI activateurs.

### EXEMPLE 2 : INHIBITION DE LA REACTIVITE D'IgG AAPC+ VIS-A-VIS DU FIBRINOGENE CITRULLINE PAR DES PEPTIDES CITRULLINES DERIVES DU FIBRINOGENE ET PORTEURS D'EPITOPES RECONNUS PAR LES AAPC

Les inventeurs ont identifié plusieurs peptides citrullinés dérivés de la fibrine porteurs d'épitopes spécifiquement reconnus par les AAPC.

Ces peptides sont listés dans le tableau 1.

Le code standard à une lettre des résidus d'acides aminés est utilisé. X indique un résidu citrullyl.

La nomenclature utilisée est la suivante : nom d'origine de la chaîne polypeptidique (α ou β) du fibrinogène dont dérive la séquence, puis position dans cette séquence du résidu amino-terminal du peptide - position du résidu carboxy-terminal du peptide. Ces positions sont numérotées par rapport à l'extrémité N-terminale des chaînes du fibrinogène (peptide signal inclus). La mention cit indique qu'il s'agit d'une forme citrullinée du peptide. La position du résidu arginyl qui est substitué par un résidu citrullyl est indiquée en indice. La nomenclature pour les formes non citrullinées des peptides est la même sauf que la mention cit et la position des résidus arginyl sont omis. Le peptide β60-74cit_{60, 72, 74} a une fonction C-terminale amidée (fonction carboxamide : CONH₂).

**TABLEAU 1**

| NOM DU PEPTIDE | SEQUENCE |
|---|---|
| α36-50cit_{38,42} | GPXVVEXHQSACKDS (SEQ ID NO: 6) |
| α171-185cit_{178,181} | VDIDIKIXSCXGSCS (SEQ ID NO: 7) |
| α183-197cit_{186,190} | SCSXALAXEVDLKDY (SEQ ID NO: 8) |
| α246-260cit₂₅₈ | PEWKALTDMPQMXME (SEQ ID NO: 9) |
| α259-273cit_{263,271} | MELEXPGGNEITXGG (SEQ ID NO: 10) |
| α366-380cit₃₆₇ | EXGSAGHWTSESSVS (SEQ ID NO: 11) |
| α396-410cit₄₀₄ | DSPGSGNAXPNNPDW (SEQ ID NO: 12) |
| α411-425cit₄₂₅ | GTFEEVSGNVSPGTX (SEQ ID NO: 13) |
| α501-515cit_{510,512} | SGIGTLDGFXHXHPD (SEQ ID NO: 14) |
| α546-560cit₅₄₇ | SXGSESGIFTNTKES (SEQ ID NO: 15) |
| α561-575cit₅₇₃ | SSHHPGIAEFPSXGK (SEQ ID NO: 16) |
| α588-602cit₅₉₁ | SYNXGDSTFESKSYK (SEQ ID NO: 17) |
| α621-635cit_{621,627,630} | XGHAKSXPVXGIHTS (SEQ ID NO: 18) |
| ß60-74cit_{60,72,74} | XPAPPPISGGGYXAX (SEQ ID NO: 19) |
| ß210-224cit₂₂₄ | QKLESDVSAQMEYCX (SEQ ID NO: 20) |
| ß281-295cit_{285,294} | VIQNXQDGSVDFGXK (SEQ ID NO: 21) |
| ß420-434cit₄₂₁ | PXKQCSKEDGGGWWY (SEQ ID NO: 22) |
| ß433-447cit_{436,445} | WYNXCHAANPNGXYY (SEQ ID NO: 23) |

Parmi ces peptides, ceux qui sont porteurs d'épitopes immunodominants (reconnus par un grand nombre de sérums de patients atteints de PR) sont présentés dans le tableau 2. Les 20 sérums testés correspondent à une série provenant de patients atteints de PR présentant des AAPC détectables par de multiples tests de détection de ces auto-anticorps et qui, pris dans leur ensemble, permettent de représenter l'hétérogénéité des profils de spécificité rencontrés chez les patients.

**TABLEAU 2**

| NOM DU PEPTIDE | NOMBRE DE SERUMS REACTIFS SUR LES 20 TESTES |
|---|---|
| ß60-74cit_{60,72,74} | 14 |
| α36-50cit_{38,42} | 12 |
| α621-635cit_{621,627,630} | 10 |
| α501-515cit_{510,512} | 9 |
| α171-185cit_{178,181} | 9 |

Le présent exemple montre la capacité de deux de ces peptides immunodominants (α36-50cit_{38, 42} et β60-74cit_{60, 72, 74}) à inhiber l'immunoréactivité des AAPC vis-à-vis du fibrinogène citrulliné humain mesurée par ELISA.

Les détails techniques de ce test de compétition par la méthode ELISA sont indiqués ci-après.

Des plaques de microtitration de 96 puits (MaxiSorp, Nunc, VWR International, Fontenay sous Bois, France) sont revêtues de fibrinogène humain citrulliné (préparé comme indiqué dans l'exemple 1) à raison de 100 µl/puit d'une solution à 5 µg/ml en PBS pH 7,4 pendant une nuit à 4°C. Les plaques sont ensuite saturées avec une solution de PBS contenant 2% de BSA (PBS-BSA 2%) (180 µl/puits). Après cette étape, sont ajoutées les IgG AAPC+ (préparées comme indiqué dans l'exemple 1), auparavant diluées à 16 µg/ml en PBS-BSA 2% en absence ou en présence de quantités croissantes des peptides compétiteurs α36-50cit_{38, 42} ou/et β60-74cit_{60, 72, 74} (chacun à une concentration variant de 0,006 à 1,56 mg/ml) ajoutés 2h avant le test ELISA. La concentration utilisée pour les IgG AAPC+ a été choisie car elle permet d'obtenir une DO entre 1 et 1,5 en absence de peptide compétiteur. Du fibrinogène citrulliné ou non citrulliné (0,0002 à 1,56 mg/ml) ainsi que les formes non citrullinées des peptides α36-50cit_{38, 42} et β60-74cit_{60, 72, 74} (respectivement dénommées α36-50 et β60-74) sont utilisés en contrôle. La fixation d'AAPC est ensuite détectée avec des IgG de chèvre anti-IgG humaines marquées à la peroxydase (SouthernBiotech, Birmingham, Alabama) diluées au 1/9000 en PBS BSA 2%. Toutes les incubations ont lieu pendant 1 h à 4°C et sont suivies de lavages en PBS-Tween-20 0,1%. L'activité de la peroxydase est révélée par une solution d'ortho-phénylènediamine (2mg/ml - Sigma-Aldrich) en peroxyde d'hydrogène (0,03% - Sigma-Aldrich) et la DO à 492 nm est mesurée à l'aide d'un lecteur de plaques (Multiskan plate reader, Thermo Labsystem, Cergy-Pontoise, France).

### Résultats:

Les résultats de ce test de compétition en ELISA sont illustrés dans la figure 2. Les données sont représentatives de 3 expériences réalisées avec deux préparations différentes d'IgG AAPC+. Les résultats sont exprimés en taux d'inhibition de la réactivité obtenue en absence de peptide compétiteur.

Le pourcentage d'inhibition de la liaison des AAPC sur le fibrinogène citrulliné fixé sur la plaque ELISA atteint une valeur plateau d'environ 80 % avec le fibrinogène citrulliné, d'environ 70% avec le mélange α36-50cit_{38,42} + β60-74cit_{60,72,74,} d'environ 60% avec β60-74cit_{60,72,74} et d'environ 15% avec α36-50cit_{38,42}. Le pourcentage d'inhibition est nul avec les même molécules non citrullinées.

Ces résultats confirment que les peptides citrullinés dérivés de la fibrine α36-50cit_{38,42} et β60-74cit_{60, 72, 74} sont porteurs d'épitopes majeurs reconnus par les AAPC puisqu'ils sont susceptibles d'induire une inhibition significative de la réactivité d'un mélange hétérogène d'AAPC vis-à-vis du fibrinogène citrulliné immobilisé au fond des plaques ELISA. Cette inhibition est majorée lorsqu'on utilise le mélange de ces deux peptides, confirmant que l'un et l'autre sont reconnus par des sous familles différentes d'AAPC.

### EXEMPLE 3 : INHIBITION DE LA STIMULATION DE MACROPHAGES HUMAINS PAR DES CI ASSOCIANT AAPC ET FIBRINOGENE CITRULLINE, A L'AIDE DE PEPTIDES CITRULLINES DERIVES DU FIBRINOGENE ET PORTEURS D'EPITOPES RECONNUS PAR DES AAPC

La préparation et la stimulation des macrophages sont réalisées exactement dans les conditions décrites dans l'exemple 1, mis à part que la reconstitution de CI est réalisée avec des IgG AAPC+ diluées à 1,13 mg/ml dans du PBS contenant 2M de NaCl et 2% de BSA en absence ou en présence du peptide compétiteur β60-74cit_{60, 72, 74} à deux concentrations (0,8 et 4 mg/ml) ajouté 2h avant l'incubation avec le fibrinogène citrulliné immobilisé au fond des plaques de culture. Des compétitions avec du fibrinogène citrulliné ou non (0,4 mg/ml), ainsi qu'avec le peptide non citrulliné β60-74 (0,8 et 4 mg/ml) sont également réalisées pour contrôle.

### Résultats:

Les résultats de ce test d'inhibition sont illustrés dans la Figure 3. Ces données sont représentatives du résultat de 2 expériences différentes conduites avec les macrophages de 2 individus différents.

L'incubation des plaques revêtues de fibrinogène non citrulliné avec les IgG AAPC+ seules ou en présence des molécules citrullinées ou non citrullinées, suivi de la stimulation des macrophages permet de définir un taux basal de TNFα inférieur à 10 pg/ml. Cette stimulation est majorée lorsque les plaques revêtues de fibrinogène citrulliné sont incubées avec les Ig AAPC+ seules ou en présence des molécules non citrullinées, et le taux de TNFa atteint des valeurs supérieures à 80 pg/ml. Cette stimulation est plus réduite lorsque les AAPC+ sont mis en présence du fibrinogène citrulliné (environ 20 pg/ml) ou du peptide β60-74Cit_{60, 72, 74} (inférieur à 20 pg/ml pour une concentration en peptide citrulliné de 4 mg/ml et environ 40 pg/ml pour une concentration en peptide citrulliné de 0,8 mg/ml)

Ces résultats montrent que le peptide β60-74Cit_{60, 72, 74} induit une inhibition dose-dépendante de la production de TNF-α en réponse aux CI. Le même résultat est observé lorsque le fibrinogène citrulliné est utilisé comme compétiteur. Par contre les formes non citrullinées du peptide et du fibrinogène n'ont pas d'effet inhibiteur.

Ces résultats indiquent que le peptide citrulliné dérivé de la fibrine β60-74cit_{60, 72, 74} est susceptible d'empêcher la formation de CI immobilisés associant AAPC et fibrinogène citrulliné. Il en résulte une inhibition de la production de TNF-α en réponse à ces CI.

## Revendications

1. Peptide citrulliné pour l'utilisation comme médicament, ledit peptide étant **caractérisé en ce qu'**il est capable de diminuer la quantité de TNF-α produit par des macrophages activés *in vitro* par des complexes immuns entre des AAPC et un antigène citrulliné reconnu par lesdits AAPC, lors de la mise en oeuvre d'un procédé selon la revendication 10, et **en ce qu'**il est choisi parmi les peptides suivants :
a) un peptide défini par la séquence X₁PAPPPISGGGYX₂AX₃ (SEQ ID NO: 1) dans laquelle X₁, X₂ représentent chacun un résidu citrullyl ou un résidu arginyl, et X₃ est un résidu citrullyl, ou un peptide de 5 à 25 acides aminés comprenant un fragment d'au moins 5 acides aminés consécutifs de ladite séquence contenant ledit résidu citrullyl X₃ ;
b) un peptide défini par la séquence GPX₁VVEX₂HQSACKDS (SEQ ID NO: 2) dans laquelle X₁ et X₂ représentent chacun un résidu citrullyl ou un résidu arginyl, et au moins l'un des résidus X₁ ou X₂ est un résidu citrullyl, ou un peptide de 5 à 25 acides aminés comprenant un fragment d'au moins 5 acides aminés consécutifs de ladite séquence contenant ledit ou lesdits résidu(s) citrullyl.

2. Peptide citrulliné pour l'utilisation comme médicament selon la revendication 1, **caractérisé en ce qu'**il est choisi dans le groupe constitué par :
- un peptide défini par la séquence SEQ ID NO: 1 dans laquelle X₁ et X₂ sont des résidus arginyl et X₃ est un résidu citrullyl, ou un peptide de 5 à 25 acides aminés comprenant un fragment d'au moins 5 acides aminés consécutifs de ladite séquence contenant ledit résidu citrullyl ;
- un peptide défini par la séquence SEQ ID NO: 1 dans laquelle X₁ est un résidu arginyl et X₂ et X₃ sont des résidus citrullyl, ou un peptide de 5 à 25 acides aminés comprenant un fragment d'au moins 5 acides aminés consécutifs de ladite séquence contenant lesdits résidus citrullyl ;
- un peptide défini par la séquence SEQ ID NO: 1 dans laquelle X₂ est un résidu arginyl et X₁ et X₃ sont des résidus citrullyl, ou un peptide de 16 à 25 acides aminés comprenant lesdits résidus citrullyl ;
- un peptide défini par la séquence SEQ ID NO: 1 dans laquelle X₁, X₂, et X₃ sont des résidus citrullyl, ou un peptide de 16 à 25 acides aminés comprenant lesdits résidus citrullyl ;
- un peptide défini par la séquence SEQ ID NO: 2 dans laquelle X₁ est un résidu arginyl et X₂ est un résidu citrullyl, ou un peptide de 5 à 25 acides aminés comprenant un fragment d'au moins 5 acides aminés consécutifs de ladite séquence contenant ledit résidu citrullyl ;
- un peptide défini par la séquence SEQ ID NO: 2 dans laquelle X₁ est un résidu citrullyl et X₂ est un résidu arginyl, ou un peptide de 5 à 25 acides aminés comprenant un fragment d'au moins 5 acides aminés consécutifs de ladite séquence contenant ledit résidu citrullyl ;
- un peptide défini par la séquence SEQ ID NO: 2 dans laquelle X₁ et X₂ sont des résidus citrullyl, ou un peptide de 5 à 25 acides aminés comprenant un fragment d'au moins 5 acides aminés consécutifs de ladite séquence contenant lesdits résidus citrullyl.

3. Peptide citrulliné pour l'utilisation comme médicament selon une quelconque des revendications 1 ou 2, **caractérisé en ce que** la fonction carboxyle (COOH) terminale dudit peptide est remplacée par une fonction carboxamide (CONH₂).

4. Peptide citrulliné pour l'utilisation comme médicament selon une quelconque des revendications 1 à 3, **caractérisé en ce qu'**il est destiné au traitement d'une maladie inflammatoire.

5. Peptide citrulliné pour l'utilisation comme médicament selon la revendication 4, **caractérisé en ce que** ladite maladie inflammatoire est la polyarthrite rhumatoïde.

6. Procédé d'évaluation *in vitro,* des propriétés de modulation de l'inflammation d'une molécule, **caractérisé en ce qu'**il comprend :
a) la mise en présence d'auto-anticorps anti-peptide citrulliné (AAPC) avec un antigène peptidique citrulliné réactif avec lesdits AAPC, dans des conditions permettant la formation de complexes immuns entre lesdits AAPC et ledit antigène citrulliné ;
b) la mise en présence des complexes immuns formés en a) avec des macrophages, dans des conditions permettant l'activation desdits macrophages par lesdits complexes immuns, et la détermination de la quantité d'au moins une cytokine pro-inflammatoire produite par lesdits macrophages ;
c) la répétition de l'étape a) et de l'étape b) l'une et/ou l'autre desdites étapes étant effectuée(s) en présence de la molécule à tester ;
d) la comparaison de la quantité de cytokine(s) pro-inflammatoire(s) produite en l'absence de la molécule à tester et en présence de celle-ci.

7. Procédé de sélection de molécules modulatrices de l'inflammation, **caractérisé en ce qu'**il comprend :
- la mise en oeuvre, avec chacune des molécules à tester, d'un procédé selon la revendication 6 ;
- la sélection des molécules capables de modifier la quantité de cytokine(s) pro-inflammatoire(s) produite(s) par les macrophages.

8. Procédé selon la revendication 7, **caractérisé en ce qu'**il s'agit d'un procédé de sélection de molécules anti-inflammatoires, et il **en ce qu'**il comprend la sélection des molécules capables de diminuer la quantité de cytokine(s) pro-inflammatoire(s) produite(s) par les macrophages.

9. Procédé selon l'une quelconque des revendications 6 à 8, **caractérisé en ce que** ledit antigène peptidique citrulliné est sélectionné dans le groupe comprenant le fibrinogène citrulliné, la filaggrine citrullinée, la vimentine citrullinée, les fragments citrullinés de ceux-ci, et leurs mélanges.

10. Procédé selon une quelconque des revendications 6 à 9, **caractérisé en ce que** la cytokine proinflammatoire quantifiée dans ledit procédé est le TNF-α.

## Claims

1. A citrullinated peptide for use as a medicament, said peptide being **characterised in that** it is capable of reducing the quantity of TNF-α produced by macrophages activated *in vitro* by immune complexes between anti-citrullinated peptide autoantibodies (ACPAs) and a citrullinated antigen recognised by said ACPAs when carrying out a method according to claim 10, and **in that** it is selected from among the following peptides:
a) a peptide defined by the sequence X₁PAPPPISGGGYX₂AX₃ (SEQ ID No. 1) in which X₁, X₂ each represent a citrullyl residue or an arginyl residue, and X₃ is a citrullyl residue, or a peptide of 5 to 25 amino acids comprising a fragment of at least 5 consecutive amino acids of said sequence containing said citrullyl residue X₃;
b) a peptide defined by the sequence GPX₁VVEX₂HQSACKDS (SEQ ID No. 2) in which X₁ and X₂ each represent a citrullyl residue or an arginyl residue, and at least one of the residues X₁ or X₂ is a citrullyl residue, or a peptide of 5 to 25 amino acids comprising a fragment of at least 5 consecutive amino acids of said sequence containing said citrullyl residue(s).

2. A citrullinated peptide for use as a medicament according to claim 1, **characterised in that** it is selected from the group made up of:
- a peptide defined by the sequence SEQ ID No. 1 in which X₁ and X₂ are arginyl residues and X₃ is a citrullyl residue, or a peptide of 5 to 25 amino acids comprising a fragment of at least 5 consecutive amino acids of said sequence containing said citrullyl residue;
- a peptide defined by the sequence SEQ ID No. 1 in which X₁ is an arginyl residue and X₂ and X₃ are citrullyl residues, or a peptide of 5 to 25 amino acids comprising a fragment of at least 5 consecutive amino acids of said sequence containing said citrullyl residues;
- a peptide defined by the sequence SEQ ID No. 1 in which X₂ is an arginyl residue and X₁ and X₃ are citrullyl residues, or a peptide of 16 to 25 amino acids comprising said citrullyl residues;
- a peptide defined by the sequence SEQ ID No. 1 in which X₁, X₂, and X₃ are citrullyl residues, or a peptide of 16 to 25 amino acids comprising said citrullyl residues;
- a peptide defined by the sequence SEQ ID No. 2 in which X₁ is an arginyl residue and X₂ is a citrullyl residue, or a peptide of 5 to 25 amino acids comprising a fragment of at least 5 consecutive amino acids of said sequence containing said citrullyl residue;
- a peptide defined by the sequence SEQ ID No. 2 in which X₁ is a citrullyl residue and X₂ is an arginyl residue, or a peptide of 5 to 25 amino acids comprising a fragment of at least 5 consecutive amino acids of said sequence containing said citrullyl residue;
- a peptide defined by the sequence SEQ ID No. 2 in which X₁ and X₂ are citrullyl residues, or a peptide of 5 to 25 amino acids comprising a fragment of at least 5 consecutive amino acids of said sequence containing said citrullyl residues.

3. A citrullinated peptide for use as a medicament according to either one of claim 1 or claim 2, **characterised in that** the terminal carboxyl function (COOH) of said peptide is replaced by a carboxamide function (CONH₂).

4. A citrullinated peptide for use as a medicament according to any one of claims 1 to 3, **characterised in that** it is intended for treating an inflammatory disease.

5. A citrullinated peptide for use as a medicament according to claim 4, **characterised in that** said inflammatory disease is rheumatoid polyarthritis.

6. An *in vitro* method for evaluating the inflammation modulatory properties of a molecule, **characterised in that** it involves:
a) bringing anti-citrullinated peptide autoantibodies (ACPAs) into contact with a citrullinated peptide antigen which reacts with said ACPAs under conditions which permit the formation of immune complexes between said ACPAs and said citrullinated antigen;
b) bringing the immune complexes formed in a) into contact with macrophages under conditions which permit the activation of said macrophages by said immune complexes, and determining the quantity of at least one proinflammatory cytokine produced by said macrophages;
c) repeating step a) and of step b), with one and/or the other of said steps being carried out in the presence of the molecule to be tested;
d) comparing the quantity of proinflammatory cytokine(s) produced in the absence of the molecule to be tested and in the presence thereof.

7. A method for selecting inflammation-modulating molecules, **characterised in that** it involves:
- carrying out a method according to claim 6 with each of the molecules to be tested;
- selecting those molecules which are capable of modifying the quantity of proinflammatory cytokine(s) produced by the macrophages.

8. A method according to claim 7, **characterised in that** it is a method for selecting antiinflammatory molecules and **in that** it involves selecting those molecules which are capable of reducing the quantity of proinflammatory cytokine(s) produced by the macrophages.

9. A method according to any one of claims 6 to 8, **characterised in that** said citrullinated peptide antigen is selected from the group comprising citrullinated fibrinogen, citrullinated filaggrin, citrullinated vimentin, citrullinated fragments thereof and mixtures thereof.

10. A method according to any one of claims 6 to 9, **characterised in that** the proinflammatory cytokine quantified in said method is TNF-α.

## Patentansprüche

1. Citrulliniertes Peptid zur Verwendung als Medikament, wobei das Peptid **dadurch gekennzeichnet ist, dass** es in der Lage ist, die Menge an TNF-α, der von Makrophagen produziert wird, die *in vitro* durch Immunkomplexe zwischen ACPA und einem von diesen ACPA erkannten citrullinierten Antigen aktiviert wurden, zu verringern, bei Durchführung eines Verfahrens nach Anspruch 10, und dadurch, dass das citrullinierte Peptid ausgewählt ist aus der Gruppe bestehend aus:
(a) einem durch die Sequenz X₁PAPPPISGGGYX₂AX₃ (SEQ ID NO: 1) definierten Peptid, worin X₁, X₂ jeweils einen Citrullylrest oder einen Arginylrest darstellen und X₃ ein Citrullylrest ist, oder einem Peptid mit 5 bis 25 Aminosäuren, das ein Fragment mit wenigstens 5 aufeinanderfolgenden Aminosäuren dieser Sequenz umfasst, das den Citrullylrest X₃ enthält;
(b) einem durch die Sequenz GPX₁VVE X₂HQSACKDS (SEQ ID NO: 2) definierten Peptid, worin X₁ und X₂ jeweils einen Citrullylrest oder einen Arginylrest darstellen und wenigstens einer der Reste X₁ oder X₂ ein Citrullylrest ist, oder einem Peptid mit 5 bis 25 Aminosäuren, das ein Fragment mit wenigstens 5 aufeinanderfolgenden Aminosäuren dieser Sequenz umfasst, das den oder die Citrullylrest(e) enthält.

2. Citrulliniertes Peptid zur Verwendung als Medikament nach Anspruch 1, **dadurch gekennzeichnet, dass** es ausgewählt ist aus der Gruppe bestehend aus:
- einem durch die Sequenz SEQ ID NO: 1 definierten Peptid, worin X₁ und X₂ Arginylreste sind und X₃ ein Citrullylrest ist, oder einem Peptid mit 5 bis 25 Aminosäuren, das ein Fragment mit wenigstens 5 aufeinanderfolgenden Aminosäuren dieser Sequenz umfasst, das den Citrullylrest enthält;
- einem durch die Sequenz SEQ ID NO: 1 definierten Peptid, worin X₁ ein Arginylrest ist und X₂ und X₃ Citrullylreste sind, oder einem Peptid mit 5 bis 25 Aminosäuren, das ein Fragment mit wenigstens 5 aufeinanderfolgenden Aminosäuren dieser Sequenz umfasst, das diese Citrullylreste enthält;
- einem durch die Sequenz SEQ ID NO: 1 definierten Peptid, worin X₂ ein Arginylrest ist und X₁ und X₃ Citrullylreste sind, oder einem Peptid mit 16 bis 25 Aminosäuren, das diese Citrullylreste enthält;
- einem durch die Sequenz SEQ ID NO: 1 definierten Peptid, worin X₁, X₂ und X₃ Citrullylreste sind, oder einem Peptid mit 16 bis 25 Aminosäuren, das diese Citrullylreste enthält;
- einem durch die Sequenz SEQ ID NO: 2 definierten Peptid, worin X₁ ein Arginylrest ist und X₂ ein Citrullylrest ist, oder einem Peptid mit 5 bis 25 Aminosäuren, das ein Fragment mit wenigstens 5 aufeinanderfolgenden Aminosäuren dieser Sequenz umfasst, das den Citrullylrest enthält;
- einem durch die Sequenz SEQ ID NO: 2 definierten Peptid, worin X₁ ein Citrullylrest ist und X₂ ein Arginylrest ist, oder einem Peptid mit 5 bis 25 Aminosäuren, das ein Fragment mit wenigstens 5 aufeinanderfolgenden Aminosäuren dieser Sequenz umfasst, das den Citrullylrest enthält;
- einem durch die Sequenz SEQ ID NO: 2 definierten Peptid, worin X₁ und X₂ Citrullylreste sind, oder einem Peptid mit 5 bis 25 Aminosäuren, das ein Fragment mit wenigstens 5 aufeinanderfolgenden Aminosäuren dieser Sequenz umfasst, das diese Citrullylreste enthält;

3. Citrulliniertes Peptid zur Verwendung als Medikament nach einem der Ansprüche 1 oder 2, **dadurch gekennzeichnet, dass** die terminale Carboxylfunktion (COOH) des Peptids durch eine Carboxamidfunktion (CONH₂) ersetzt ist.

4. Citrulliniertes Peptid zur Verwendung als Medikament nach einem der Ansprüche 1 bis 3, **dadurch gekennzeichnet, dass** es zur Behandlung einer entzündlichen Erkrankung vorgesehen ist.

5. Citrulliniertes Peptid zur Verwendung als Medikament nach Anspruch 4, **dadurch gekennzeichnet, dass** die entzündliche Erkrankung rheumatoide Arthritis ist.

6. In-vitro-Verfahren zum Ermitteln der entzündungsmodulierenden Eigenschaften eines Moleküls, **dadurch gekennzeichnet, dass** es umfasst:
a) In-Kontakt-bringen von Autoantikörpern gegen citrullinierte Peptide (ACPA) mit einem mit diesen ACPA reagierenden citrullinierten Peptidantigen unter Bedingungen, die die Bildung von Immunkomplexen zwischen diesen ACPA und dem citrullinierten Antigen erlauben;
b) In-Kontakt-bringen der in a) gebildeten Immunkomplexe mit Makrophagen unter Bedingungen, die die Aktivierung der Makrophagen durch die Immunkomplexe erlauben, und Bestimmen der Menge wenigstens eines von den Makrophagen produzierten proinflammatorischen Zytokins;
c) Wiederholen von Schritt a) und Schritt b), wobei der eine und/oder der andere dieser Schritte in Gegenwart des zu testenden Moleküls durchgeführt wird;
d) Vergleichen der in Abwesenheit des zu testenden Moleküls und in dessen Gegenwart produzierten Menge an proinflammatorischem (proinflammatorischen) Zytokin(en).

7. Selektionsverfahren für entzündungsmodulierende Moleküle, **dadurch gekennzeichnet, dass** es umfasst:
- Durchführen eines Verfahrens nach Anspruch 6 mit jedem der zu testenden Moleküle;
- Selektion von Molekülen, die in der Lage sind, die Menge an durch die Makrophagen produziertem (produzierten) proinflammatorischen Zytokin(en) zu modifizieren.

8. Verfahren nach Anspruch 7, **dadurch gekennzeichnet, dass** es sich um ein Selektionsverfahren für antiinflammatorische Moleküle handelt, und dadurch, dass es die Selektion von Molekülen umfasst, die in der Lage sind, die Menge an durch die Makrophagen produziertem (produzierten) proinflammatorischen Zytokin(en) zu verringern.

9. Verfahren nach einem der Ansprüche 6 bis 8, **dadurch gekennzeichnet, dass** das citrullinierte Peptidantigen ausgewählt ist aus der Gruppe umfassend citrulliniertes Fibrinogen, citrulliniertes Filaggrin, citrulliniertes Vimentin, citrullinierte Fragmente davon und Mischungen davon.

10. Verfahren nach einem der Ansprüche 6 bis 9, **dadurch gekennzeichnet, dass** das bei diesem Verfahren quantifizierte proinflammatorische Zytokin TNF-α ist.


### SEQUENCE LISTING

<110> UNIVERSITE PAUL SABATIER
   CLAVEL, Cyril
   SEBBAG, Mireille
   NOGUEIRA, Maria Leonor
   SERRE, Guy
<120> Peptides modulateurs de l'activation des macrophages, utilisables pour le traitement de la polyarthrite rhumatoïde.
<130> MJP/mad-F1249/4-WO
<150> FR 06/03956
   <151> 2006-05-03
<160> 23
<170> PatentIn version 3.3
<210> 1
   <211> 15
   <212> PRT
   <213> Artificial
<220>
   <223> peptide citrulliné
<220>
   <221> MISC_FEATURE
   <222> (1) .. (1)
   <223> X = résidu citrullyl ou arginyl
<220>
   <221> MISC_FEATURE
   <222> (13) .. (13)
   <223> X = résidu citrullyl ou arginyl
<220>
   <221> MISC_FEATURE
   <222> (15)..(15)
   <223> X = résidu citrullyl ou arginyl
<400> 1
   Xaa Pro Ala Pro Pro Pro Ile Ser Gly Gly Gly Tyr Xaa Ala Xaa
   1               5                   10                  15
<210> 2
   <211> 15
   <212> PRT
   <213> Artificial
<220>
   <223> peptide citrulliné
<220>
   <221> MISC_FEATURE
   <222> (3)..(3)
   <223> X = résidu citrullyl ou arginyl
<220>
   <221> MISC_FEATURE
   <222> (7) .. (7)
   <223> X = résidu citrullyl ou arginyl
<400> 2
   Gly Pro Xaa Val Val Glu Xaa His Gln Ser Ala Cys Lys Asp Ser
   1               5                   10                  15
<210> 3
   <211> 15
   <212> PRT
   <213> Artificial
<220>
   <223> peptide citrulliné
<220>
   <221> MISC_FEATURE
   <222> (10) .. (10)
   <223> X = résidu citrullyl ou arginyl
<220>
   <221> MISC_FEATURE
   <222> (12) .. (12)
   <223> X = résidu citrullyl ou arginyl
<400> 3
   Ser Gly Ile Gly Thr Leu Asp Gly Phe Xaa His Xaa His Pro Asp
   1               5                   10                  15
<210> 4
   <211> 15
   <212> PRT
   <213> Artificial
<220>
   <223> peptide citrulliné
<220>
   <221> MISC_FEATURE
   <222> (8) .. (8)
   <223> X = résidu citrullyl ou arginyl
<220>
   <221> MISC_FEATURE
   <222> (11) .. (11)
   <223> X = résidu citrullyl ou arginyl
<400> 4
   Val Asp Ile Asp Ile Lys Ile Xaa Ser Cys Xaa Gly Ser Cys Ser
   1               5                   10                  15
<210> 5
   <211> 15
   <212> PRT
   <213> Artificial
<220>
   <223> peptide citrulliné
<220>
   <221> MISC_FEATURE
   <222> (1) .. (1)
   <223> X = résidu citrullyl ou arginyl
<220>
   <221> MISC_FEATURE
   <222> (7) .. (7)
   <223> X = résidu citrullyl ou arginyl
<220>
   <221> MISC_FEATURE
   <222> (10) .. (10)
   <223> X = résidu citrullyl ou arginyl
<400> 5
   Xaa Gly His Ala Lys Ser Xaa Pro Val Xaa Gly Ile His Thr Ser
   1               5                   10                  15
<210> 6
   <211> 15
   <212> PRT
   <213> Artificial
<220>
   <223> peptide citrulliné
<220>
   <221> MISC_FEATURE
   <222> (3) .. (3)
   <223> X = résidu citrullyl
<220>
   <221> MISC_FEATURE
   <222> (7) .. (7)
   <223> X = résidu citrullyl
<400> 6
   Gly Pro Xaa Val Val Glu Xaa His Gln Ser Ala Cys Lys Asp Ser
   1               5                   10                  15
<210> 7
   <211> 15
   <212> PRT
   <213> Artificial
<220>
   <223> peptide citrulliné
<220>
   <221> MISC_FEATURE
   <222> (10) .. (10)
   <223> X = résidu citrullyl
<220>
   <221> MISC_FEATURE
   <222> (12) .. (12)
   <223> X = résidu citrullyl
<400> 7
   Ser Gly Ile Gly Thr Leu Asp Gly Phe Xaa His Xaa His Pro Asp
   1               5                   10                  15
<210> 8
   <211> 15
   <212> PRT
   <213> Artificial
<220>
   <223> peptide citrulliné
<220>
   <221> MISC_FEATURE
   <222> (8) .. (8)
   <223> X = résidu citrullyl
<220>
   <221> MISC_FEATURE
   <222> (11) .. (11)
   <223> X = résidu citrullyl
<400> 8
   Val Asp Ile Asp Ile Lys Ile Xaa Ser Cys Xaa Gly Ser Cys Ser
   1               5                   10                  15
<210> 9
   <211> 15
   <212> PRT
   <213> Artificial
<220>
   <223> peptide citrulliné
<220>
   <221> MISC_FEATURE
   <222> (1) .. (1)
   <223> X = résidu citrullyl
<220>
   <221> MISC_FEATURE
   <222> (7) .. (7)
   <223> X = résidu citrullyl
<220>
   <221> MISC_FEATURE
   <222> (10) .. (10)
   <223> X = résidu citrullyl
<400> 9
   Xaa Gly His Ala Lys Ser Xaa Pro Val Xaa Gly Ile His Thr Ser
   1               5                   10                  15
<210> 10
   <211> 15
   <212> PRT
   <213> Artificial
<220>
   <223> peptide citrulliné
<220>
   <221> MISC_FEATURE
   <222> (4) .. (4)
   <223> X = résidu citrullyl
<220>
   <221> MISC_FEATURE
   <222> (8) .. (8)
   <223> X = résidu citrullyl
<400> 10
   Ser Cys Ser Xaa Ala Leu Ala Xaa Glu Val Asp Leu Lys Asp Tyr
   1               5                   10                  15
<210> 11
   <211> 15
   <212> PRT
   <213> Artificial
<220>
   <223> peptide citrulliné
<220>
   <221> MISC_FEATURE
   <222> (13) .. (13)
   <223> X = résidu citrullyl
<400> 11
   Pro Glu Trp Lys Ala Leu Thr Asp Met Pro Gln Met Xaa Met Glu
   1               5                   10                  15
<210> 12
   <211> 15
   <212> PRT
   <213> Artificial
<220>
   <223> peptide citrulliné
<220>
   <221> MISC_FEATURE
   <222> (5) .. (5)
   <223> X = résidu citrullyl
<220>
   <221> MISC_FEATURE
   <222> (13) .. (13)
   <223> X = résidu citrullyl
<400> 12
   Met Glu Leu Glu Xaa Pro Gly Gly Asn Glu Ile Thr Xaa Gly Gly
   1               5                   10                  15
<210> 13
   <211> 15
   <212> PRT
   <213> Artificial
<220>
   <223> peptide citrulliné
<220>
   <221> MISC_FEATURE
   <222> (2) .. (2)
   <223> X = résidu citrullyl
<400> 13
   Glu Xaa Gly Ser Ala Gly His Trp Thr Ser Glu Ser Ser Val Ser
   1               5                   10                  15
<210> 14
   <211> 15
   <212> PRT
   <213> Artificial
<220>
   <223> peptide citrulliné
<220>
   <221> MISC_FEATURE
   <222> (9) .. (9)
   <223> X = résidu citrullyl
<400> 14
   Asp Ser Pro Gly Ser Gly Asn Ala Xaa Pro Asn Asn Pro Asp Trp
   1               5                   10                  15
<210> 15
   <211> 15
   <212> PRT
   <213> Artificial
<220>
   <223> peptide citrulliné
<220>
   <221> MISC_FEATURE
   <222> (15) .. (15)
   <223> X = résidu citrullyl
<400> 15
   Gly Thr Phe Glu Glu Val Ser Gly Asn Val Ser Pro Gly Thr Xaa
   1 5 10 15
<210> 16
   <211> 15
   <212> PRT
   <213> Artificial
<220>
   <223> peptide citrulliné
<220>
   <221> MISC_FEATURE
   <222> (2) .. (2)
   <223> X = résidu citrullyl
<400> 16
   Ser Xaa Gly Ser Glu Ser Gly Ile Phe Thr Asn Thr Lys Glu Ser
   1 5 10 15
<210> 17
   <211> 15
   <212> PRT
   <213> Artificial
<220>
   <223> peptide citrulliné
<220>
   <221> MISC_FEATURE
   <222> (13) .. (13)
   <223> X = résidu citrullyl
<400> 17
   Ser Ser His His Pro Gly Ile Ala Glu Phe Pro Ser Xaa Gly Lys
   1 5 10 15
<210> 18
   <211> 15
   <212> PRT
   <213> Artificial
<220>
   <223> peptide citrulliné
<220>
   <221> MISC_FEATURE
   <222> (4) .. (4)
   <223> X = résidu citrullyl
<400> 18
   Ser Tyr Asn Xaa Gly Asp Ser Thr Phe Glu Ser Lys Ser Tyr Lys
   1               5                   10                  15
<210> 19
   <211> 15
   <212> PRT
   <213> Artificial
<220>
   <223> peptide citrulliné
<220>
   <221> MISC_FEATURE
   <222> (1) .. (1)
   <223> X = résidu citrullyl
<220>
   <221> MISC_FEATURE
   <222> (13) .. (13)
   <223> X = résidu citrullyl
<220>
   <221> MISC_FEATURE
   <222> (15) .. (15)
   <223> X = résidu citrullyl
<400> 19
   Xaa Pro Ala Pro Pro Pro Ile Ser Gly Gly Gly Tyr Xaa Ala Xaa
   1               5                   10                  15
<210> 20
   <211> 15
   <212> PRT
   <213> Artificial
<220>
   <223> peptide citrulliné
<220>
   <221> MISC_FEATURE
   <222> (15) .. (15)
   <223> X = résidu citrullyl
<400> 20
   Gln Lys Leu Glu Ser Asp Val Ser Ala Gln Met Glu Tyr Cys Xaa
   1               5                   10                  15
<210> 21
   <211> 15
   <212> PRT
   <213> Artificial
<220>
   <223> peptide citrulliné
<220>
   <221> MISC_FEATURE
   <222> (5) .. (5)
   <223> X = résidu citrullyl
<220>
   <221> MISC_FEATURE
   <222> (14) .. (14)
   <223> X = résidu citrullyl
<400> 21
   Val Ile Gln Asn Xaa Gln Asp Gly Ser Val Asp Phe Gly Xaa Lys
   1               5                   10                  15
<210> 22
   <211> 15
   <212> PRT
   <213> Artificial
<220>
   <223> peptide citrulliné
<220>
   <221> MISC_FEATURE
   <222> (2) .. (2)
   <223> X = résidu citrullyl
<400> 22
   Pro Xaa Lys Gln Cys Ser Lys Glu Asp Gly Gly Gly Trp Trp Tyr
   1               5                   10                  15
<210> 23
   <211> 15
   <212> PRT
   <213> Artificial
<220>
   <223> peptide citrulliné
<220>
   <221> MISC_FEATURE
   <222> (4) .. (4)
   <223> X = résidu citrullyl
<220>
   <221> MISC_FEATURE
   <222> (13) .. (13)
   <223> X = résidu citrullyl
<400> 23
   Trp Tyr Asn Xaa Cys His Ala Ala Asn Pro Asn Gly Xaa Tyr Tyr
   1 5 10 15